# EUROPÄISCHE PATENTSCHRIFT

(11) **EP 3 560 452 B1**
(45) Veröffentlichungstag und Bekanntmachung des Hinweises auf die Patenterteilung: **15.05.2024**
(21) Anmeldenummer: 18168896.1
(22) Anmeldetag: 24.04.2018
(51) Int. Cl.: A61B 90/00, A61F 2/82

(54) **MARKERELEMENT UND VERFAHREN ZU DESSEN HERSTELLUNG**
MARKER ELEMENT AND METHOD FOR ITS PRODUCTION
ÉLÉMENT MARQUEUR ET SON PROCÉDÉ DE FABRICATION

(43) Veröffentlichungstag der Anmeldung: 30.10.2019
(73) Patentinhaber: BIOTRONIK AG, 8180 Bülach (CH)
(72) Erfinder: Risch, Fabian, 8200 Schaffhausen (CH)
(74) Vertreter: Biotronik Corporate Services SE

(56) Entgegenhaltungen:
- EP-A1- 1 570 808
- EP-A1- 2 526 901
- EP-A1- 3 281 648
- EP-A2- 2 399 619
- WO-A1-2007/105067
- WO-A1-2016/201317
- US-A1- 2006 235 505

## Beschreibung

Die vorliegende Erfindung bezieht sich auf ein Markerelement für ein Implantat und ein Gerüst für ein Implantat mit einem Markerelement sowie ein Verfahren zur Herstellung eines solchen Markerelements und eines solchen Gerüsts.

Die vorliegende Erfindung betrifft außerdem ein Implantat, insbesondere eine intraluminale Endoprothese, mit einem Gerüst (Scaffold) und mit einem an dem Gerüst befestigten Markerelement, das zumindest in einem Teil seines Volumens eine verglichen mit dem Material des Gerüsts verschiedene Materialzusammensetzung aufweist, die radioopakes und/oder röntgenopakes Material umfasst, sowie ein Verfahren zur Herstellung eines solchen Implantats.

Die Implantate sind endovaskuläre Prothesen (Endoprothesen, Stents) oder andere Implantate, die zur Behandlung von Stenosen (Gefäßverengungen) eingesetzt werden können. Sie weisen meist ein Gerüst in Form eines hohlzylinder- oder röhrenförmigen Grundgitters auf, das an beiden Längsenden der Röhren offen ist. Ein solches Gerüst weist meist eine Vielzahl von miteinander verbundenen Streben (Struts) auf, die das Grundgitter ausbilden. Das röhrenförmige Grundgitter einer derartigen Endoprothese wird in das zu behandelnde Gefäß eingesetzt und dient dazu, das Gefäß zu stützen. Weitere Gerüstformen sind ebenfalls möglich. Ferner betrifft die vorliegende Erfindung Implantate, welche im Bereich der Orthopädie verwendet werden können, z.B. für den Schädelbereich, und insbesondere Implantate, die auf Grund ihrer geringen Größe und Wanddicke eine geringe Röntgensichtbarkeit aufweisen. Die Erfindung kann ebenfalls für Stents im neurovaskulären Bereich zur Anwendung kommen. Hier kommt es darauf an, die hirnversorgenden Blutgefäße mit absorbierbaren Mg-Stents offen zu halten. Diese Systeme kommen auf dem Gebiet der Verhinderung akuter ischämischer Schlaganfälle zum Einsatz.

Stents oder andere Implantate weisen in ihrem Gerüst häufig metallische Materialien auf. Hierbei können die metallischen Materialien einen biodegradierbaren Werkstoff bilden, wobei auch polymere biodegradierbare Materialien enthalten sein können.

Unter Biodegradation werden hydrolytische, enzymatische und andere stoffwechselbedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile des Gerüsts bzw. des Implantats führen. Synonym zu dem Begriff Biodegradation wird häufig der Begriff Biokorrosion verwendet. Der Begriff Bioresorption umfasst die anschließende Resorption der Abbauprodukte durch den lebenden Organismus. Das Ziel der Verwendung biodegradierbarer Implantate besteht darin, dass sie zu einem Zeitpunkt, wenn sie beispielsweise in Bezug auf ihre Stützwirkung nicht mehr benötigt werden, vom Organismus abgebaut sind und demzufolge nicht länger als nötig als Fremdkörper im Organismus vorhanden sind.

Für das Gerüst biodegradierbarer Implantate geeignete Werkstoffe (Grundmaterial) können aus einem Material oder mehreren Materialien bestehen. Beispiele für geeignete polymere Verbindungen sind Polymere aus der Gruppe Cellulose, Kollagen, Albumin, Casein, Polysaccharide (PSAC), Polylactid (PLA), Poly-LLactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren auf Legierungen von Magnesium, Eisen, Zink und/oder Wolfram.

Die Ermittlung der Position eines Stents oder anderer Implantate geschieht häufig mittels bildgebender Verfahren, beispielsweise mittels einer Röntgenstrahleinrichtung. Aufgrund der kleinen Ordnungszahl und der geringen Dichte des biodegradierbaren Materials, z.B. Magnesium und seiner Legierungen, ist die Röntgensichtbarkeit der daraus gefertigten medizinischen Implantate sehr gering. Um diesen Nachteil zu beheben, ist es bekannt, medizinische Vorrichtungen mit Markerelementen zu versehen, die zumindest in einem Teil ihres Volumens eine verglichen mit dem Material des Gerüsts verschiedene Materialzusammensetzung aufweisen. Diese sogenannten (Röntgen-)Marker oder Markerelemente enthalten insbesondere ein Material, das die Röntgenstrahlen und/oder andere elektromagnetische Strahlen stärker absorbiert (im Folgenden als röntgenopakes oder radioopakes Material bezeichnet) als das Material des Gerüsts bzw. die Körperumgebung des Patienten und hierdurch relativ zu seiner Umgebung sichtbar wird. Basierend auf der ermittelten Lage der meist mehreren Markerelemente an dem Gerüst lässt sich die Lage und Winkelposition des Implantats in Bezug auf die umgebenden Organe bestimmen. Bei Verwendung eines biodegradierbaren Gerüsts wird aus Gründen der ausreichenden Röntgensichtbarkeit häufig ein nicht resorbierbares röntgenopakes oder radioopakes Material (z.B. Ta, Au, W) eingesetzt.

Ein solches Markerelement wird häufig aus einem Halbzeug bestehend aus dem Material des Markerelements ab- oder ausgeschnitten und derart in ein Implantat integriert, dass es in eine entsprechende Öffnung (Eyelet), die hierfür am Gerüst des Implantats vorgesehen ist (z.B. an beiden Enden in axialer Richtung des Implantats), eingeklebt wird. Derartige Markerelemente und Implantate sind beispielsweise aus den Druckschriften EP 3 281 648 A1, EP 2 399 619 B1, EP 3 165 238 A1 und EP Anmeldung Nr. 17150973.0, am 18.07.2018 als EP 3 348 239 A1 veröffentlicht, bekannt.

Es besteht die technische Anforderung an Implantate mit integrierten Markerelementen, dass die Markerelemente über einen langen Zeitraum mit einer ausreichenden Adhäsionskraft im Gerüstverbund verbleiben. Bei einer nicht ausreichenden Anbindung des Markerelements an das Gerüst des Implantats kann sich durch einen metallischen Kontakt zwischen Markerelement und Gerüstmaterial ein unerwünschtes Lokalelement ausbilden oder Kontaktkorrosion verursacht werden. Dies würde zu einer frühzeitigen Abtrennung des Markerelements von dem Gerüst des Implantats führen, was eine nicht gewünschte Fragmentbildung und Embolisation begünstigen würde. Zudem soll generell verhindert werden, dass das Material des Markerelements einen Einfluss auf die Degradation des Gerüsts hat. Zudem ist bei den herkömmlichen Klebeverfahren die Dosierung des Klebstoffs schwierig, da für jedes Markerelement sehr kleine Klebstoffmengen verwendet werden. Auch die Positionierung des für das Einkleben benötigten Klebstoffs ist diffizil und daher häufig ungenau.

Die Aufgabe der vorliegenden Erfindung besteht somit darin, ein Implantat bzw. ein Gerüst für ein Implantat zu schaffen, bei dem eine Lokalelementbildung sowie Korrosion zumindest reduziert wird. Entsprechend sollte ein Markerelement geschaffen werden, das nach der Integration in das Implantat kein Lokalelement bildet und Korrosion vermeidet. Weiter sollen kostengünstige, einfache und automatisierbare Verfahren zur Herstellung eines derartigen Markerelements, eines Gerüsts mit einem solchen integrierten Markerelement oder eines Implants mit einem derartigen Gerüst angegeben werden. Hierbei sollte insbesondere das Klebstoff-Handling verbessert werden.

Die Aufgabe wird gelöst durch das in Anspruch 1 angegebenen Verfahren zur Herstellung eines Markerelements.

Die Aufgabe wird insbesondere gelöst durch ein Verfahren zur Herstellung eines Markerelements für ein Implantat aus einem schichtförmigen oder drahtförmigen Halbzeug aufweisend ein röntgenopakes oder radioopakes Material, wobei auf oder an mindestens einer Seite des Halbzeugs oder einer Vielzahl von Abschnitten des Halbzeugs jeweils eine Klebstoff-Schicht befestigt wird, so dass zumindest abschnittsweise ein Schichtverbund gebildet wird, und wobei anschließend eine Vielzahl von Markerelementen durch einen entsprechenden Schnitt oder eine entsprechende Durchtrennung (z.B. Durchbrechen eines Stegs) in eine Richtung quer, vorzugsweise senkrecht, zu den Schichten des Schichtverbunds aus dem Schichtverbund ausgeschnitten oder von dem Schichtverbund abgetrennt werden. Das Befestigen der Klebstoff-Schicht auf dem Halbzeug kann beispielsweise mittels Beschichten (z.B. Dipcoating oder Spraycoating) erfolgen. Es ist eine oder mehrere weitere Schicht aufweisend ein röntgenopakes oder radioopakes Material im Schichtverbund vorgesehen.

Das erfindungsgemäße Verfahren hat den Vorteil, dass eine Klebstoffdosierung auf einzelne Markerelemente entfällt. Mittels eines automatisierbaren Verfahrens können effektiv Markerelemente hergestellt werden, die bereits eine für die jeweilige Anwendung genau dosierte und ideal positionierte Klebstoff-Schicht aufweisen.

Alternativ kann eine Beschichtung der einzelnen Markerelemente im Schüttgut erfolgen.

Das so entstandene Markerelement kann dann in eine Öffnung (Eyelet) des Gerüsts eingesetzt und mit diesem verklebt werden. Gegebenenfalls finden vor dem Abtrennen des Markerelements von dem Schichtverbund weitere Behandlungen und/oder Beschichtungen des Schichtverbunds statt (siehe unten).

Das schichtförmige Halbzeug wird beispielsweise durch eine Folie, eine Platte, einen Hohlzylinder (Rohr) oder ein dreiseitiges, vierseitiges oder mehr als vierseitiges, vorzugsweise gerades Hohlprisma, das an beiden Enden in Richtung einer Längsachse offen ist, gebildet.

Das Halbzeug weist ein röntgenopakes oder radioopakes Material in der Form eines Metalls oder einer Metalllegierung enthaltend mindestens ein Metall oder Edelmetall ausgewählt aus der Gruppe mit den Elementen Gold, Platin, Wolfram, Tantal, und Titan auf oder besteht aus einem solchen Metall oder einer solchen Metalllegierung. Besonders bevorzugt besteht das Halbzeug aus Tantal oder einer Tantallegierung. Die angegebenen Materialen des Markerelements besitzen gute Röntgenabsorptionseigenschaften und lassen sich, z.B. durch plasmaelektrolytische Oxidation oder andere Passivierungsverfahren (wie beispielsweise die Verwendung einer passivierenden Beschichtung mit einem Polymer oder Parylene bei Verwendung von Gold als radioopakes Materials), an ihrer Oberfläche sehr einfach mit einer dichten, passivierenden und isolierenden (d.h. elektrisch nicht leitenden) Oxidschicht versehen. Sie erlauben hierdurch eine Gestaltung eines Markerelements mit minimaler Größe. Vorzugsweise wird das Halbzeug durch Ziehen aus dem jeweiligen Material hergestellt.

Das Abtrennen des mindestens einen Abschnitts von dem Halbzeug erfolgt (beispielsweise nach einer Reinigung (Beizen) und/oder Passivierungsbehandlung) mittels Durchschneiden (z.B. mittels Laser), Durchbrechen eines Stegs oder mechanischem Abschneiden von dem Halbzeug. Gegebenenfalls kann der Abschnitt aus dem Halbzeug entlang eines Teils der Kontur abgetrennt werden, so dass der Abschnitt noch über mindestens einen Steg mit dem Halbzeug verbunden ist. Danach kann eine Reinigung (Beizen) und/oder eine Passivierungsbehandlung erfolgen. Anschließend wird mittels Durchtrennen (z.B. mittels Laser oder mittels eines mechanischen Schneidwerkzeugs) des mindestens einen Stegs der Abschnitt vollständig von dem Halbzeug abgetrennt.

Die Klebstoff-Schicht weist vorzugsweise ein thermoplastisches Elastomer (TPE) auf oder besteht aus einem TPE. TPEs (gelegentlich auch Elastoplaste genannt) sind Kunststoffe, die sich bei Raumtemperatur vergleichbar den klassischen Elastomeren verhalten, sich jedoch unter Wärmezufuhr plastisch verformen lassen und somit ein thermoplastisches Verhalten zeigen. TPE umfasst beispielsweise die Materialklassen
- PE-A oder TPA (Thermoplastische Copolyamide, z. B. PEBAX (Arkema)),
- TPE-E oder TPC (Thermoplastische Polyesterelastomere / Thermoplastische Copolyester, z. B. Keyflex (LG Chem)),
- TPE-O oder TPO (Thermoplastische Elastomere auf Olefinbasis, vorwiegend PP/EPDM),
- TPE-S oder TPS (Styrol-Blockcopolymere (SBS, SEBS, SEPS, SEEPS und MBS), z. B. Kraton (Kraton Polymers), Septon (Kuraray), Styroflex (BASF), Thermolast (Kraiburg TPE) oder Saxomer (PCW)),
- TPE-U oder TPU (Thermoplastische Elastomere auf Urethanbasis, z. B. Elastollan (BASF) oder Desmopan, Texin, Utechllan (Bayer)) und
- TPE-V oder TPV (Thermoplastische Vulkanisate oder vernetzte thermoplastische Elastomere auf Olefinbasis, vorwiegend PP/EPDM, z. B. Sarlink (DSM)).

Elastische Klebstoffe haben den Vorteil, dass sie zu einer verbesserten Trackability, also zur Anpassung an das umgebende Gewebe während des Vordringens des Implantats beim Einsetzen, beitragen, sodass ein vorzeitiger Verlust eines Markerelements vermieden wird.

Beispielsweise kann als TPE-Klebstoff Polyurethan gelöst in Dimethylformamid verwendet werden. Alternative Klebestoffe aus den obigen Materialklassen sind ebenfalls denkbar, vorzugsweise mit einem geeigneten Lösungsmittel, das unter Wärmeeinwirkung verdampft, um ein Aushärten des Klebstoffs zu bewirken.

Alternativ oder zusätzlich kann die Klebstoff-Schicht ein Harz und/oder Schelllack und/oder einen niedrigviskosen Klebstoff aufweisen.

Als vorteilhafter polymerbasierter Klebstoff kann neben Polyurethan beispielsweise ein degradierbares Polymer (z. B. PLLA L210, PLLA L214) verwendet werden. Diese Klebstoffe sind besonders bioverträglich und außerdem gut elektrisch isolierend.

In einem Ausführungsbeispiel werden bei dem Verfahren zur Herstellung des Markerelements mit einem schichtförmigen (d.h. folienartigen, plattenförmigen oder hohlkörperförmigen) Halbzeug
- eine durchgehende erste Schicht oder eine Vielzahl von Abschnitten einer ersten Schicht des Halbzeugs auf einer ersten Seite der Klebstoff-Schicht sowie
- eine durchgehende zweite Schicht oder eine Vielzahl von Abschnitten einer zweiten Schicht des Halbzeugs auf einer zweiten, der ersten Seite gegenüber liegenden Seite der Klebstoff-Schicht
angeordnet und an dieser befestigt, so dass diese zumindest abschnittsweise den Schichtverbund bilden.

Durch das in diesem Ausführungsbeispiel beschriebene Verfahren wird ein Markerelement mit einem Sandwich-Design geschaffen, das beim Einkleben in eine Öffnung des Gerüsts die unten beschriebenen Vorteile besitzt.

Das obige Ausführungsbeispiel eines Verfahrens zur Herstellung eines Markerelements lässt sich insbesondere gut automatisieren, wenn eine durchgehende erste Schicht eines folienartigen Halbzeugs aufweisend ein röntgenopakes oder radioopakes Material auf einer ersten Seite der Klebstoff-Schicht sowie eine durchgehende zweite Schicht eines folienartigen Halbzeugs aufweisend ein röntgenopakes oder radioopakes Material auf der zweiten Seite der Klebstoff-Schicht, die der ersten Seite gegenüber liegt, angeordnet und an dieser befestigt wird, so dass diese zumindest abschnittsweise den Schichtverbund bilden.

Die obige Aufgabenstellung wird außerdem durch ein Verfahren zur Herstellung eines Gerüsts, bevorzugt aus einer Magnesiumlegierung, insbesondere aus einer seltene Erden enthaltenden Magnesiumlegierung, einer Magnesium-Zink-Aluminium Legierung oder einer Magnesium-Zink-Calcium Legierung, für ein Implantat mit einem Markerelement aufweisend einen Schichtverbund mit mindestens einer Schicht aufweisend ein röntgenopakes oder radioopakes Material und einer Klebstoff-Schicht gelöst, bei dem das Markerelement in eine Öffnung des Gerüsts eingebracht und mittels einer Wärmequelle das Markerelement derart erwärmt wird, dass die Klebstoff-Schicht des Markerelements erweicht oder verflüssigt wird, sodass der Klebstoff der Klebstoff-Schicht eine Klebstoff-Verbindung mit der Innenfläche der Öffnung bewirkt.

Bei dem erfindungsgemäßen Verfahren besitzt das Markerelement vorzugsweise eine von dem Material des Gerüsts verschiedene Zusammensetzung.

Erfindungsgemäß wird das mindestens eine Markerelement mit einem Klebstoff, vorzugsweise mit einem Klebstoff mit oder bestehend aus TPE, an dem Gerüst befestigt. Es wurde nämlich erkannt, dass insbesondere durch eine stoffschlüssige Verbindung eine einfache und mechanische, das filigrane Gerüst nicht belastende Verbindung erzielt wird. Zusätzlich kann durch Anpassung der Form des Markerelements an die Form der Öffnung (Eyelet, Aufnahme) am Gerüst des Implantats, in welche das Markerelement eingebracht wird, oder umgekehrt ein Formschluss realisiert werden. Hierbei sind jedoch die inneren Abmessungen (z.B. die innere Breite und Länge) der Öffnung ein wenig größer als die äußeren Abmessungen des Markerelements, so dass um den äußeren Rand des Markerelements nach dem Einbringen in die Öffnung noch ein Spalt zwischen Markerelement und Innenrand der Öffnung verbleibt, in den der Klebstoff nach dem Erweichen bzw. Verflüssigen fließen kann, um eine stoffschlüssige Verbindung zwischen Markerelement und Öffnung herzustellen.

In einem Ausführungsbeispiel ist die Öffnung durchgehend gestaltet.

In einer Weiterbildung der Erfindung wird bei Verwendung eines Markerelements aufweisend einen Schichtverbund mit mindestens einer ersten Schicht und einer zweiten Schicht, jeweils aufweisend ein röntgenopakes oder radioopakes Material, und einer dazwischen liegenden Klebstoff-Schicht, das Markerelement nach dem Einbringen in eine Öffnung des Gerüsts derart mit einer Druckkraft in eine Richtung quer zu den Schichten des Schichtverbunds, vorzugsweise senkrecht zu den Schichten des Schichtverbunds, beaufschlagt, dass der Klebstoff an der Seitenfläche des Markerelements austritt und eine Klebstoff-Verbindung mit der Innenfläche der Öffnung bewirkt. Die Druckkraft kann beispielsweise mittels einer entsprechenden Crimpzange oder Crimpwerkzeug aufgebracht werden und bewirkt die Verteilung des Klebstoffs in dem Spalt zwischen Markerelement und Innenrand der Öffnung. Das Zusammenpressen der ersten Schicht und der zweiten Schicht mit dem röntgenopaken oder radioopaken Material verringert den Platz für den Klebstoff zwischen diesen Schichten und verursacht ein seitliches Herausquellen des Klebstoffs aus dem Markerelement in den Klebespalt zwischen Markerelement und Öffnung. Bei einem hohlzylindrischen Gerüst verläuft die Druckkraft vorzugsweise in radialer Richtung. Dieses Ausführungsbeispiel ist besonders gut für eine weitere Automatisierung des Verfahrens geeignet und erzielt besonders gute Ergebnisse hinsichtlich der Verbindung mit dem Gerüst und der Vermeidung der Lokalelementbildung. In vorteilhafter Weise ist bei diesem Ausführungsbeispiel keine Kapillarwirkung notwendig, um den Klebstoff im Klebespalt zu verteilen.

In einem bevorzugten Ausführungsbeispiel wird das Gerüst vor dem Einbringen des Markerelements in die Öffnung auf einen Dorn gesteckt, der das innere Volumen des Gerüsts ausfüllt. Bei einem hohlzylinderförmigen Gerüst kann der Dorn beispielsweise die Form eines Zylinders aufweisen. Der Dorn bildet ein Widerlager für das Aufbringen der Drucckraft und kann nach dem Ankleben des mindestens einen Markerelements an das Gerüst wieder entfernt werden.

In einem weiteren Ausführungsbeispiel wird das Markerelement vor oder während der Beaufschlagung des Markerelements mit der Druckkraft durch die Wärmequelle derart erwärmt, dass die Klebstoff-Schicht des Markerelements erweicht oder verflüssigt wird. Bei diesem Ausführungsbeispiel wird das seitliche Austreten (Herausquellen) des Klebstoffs aus dem Sandwich-Schichtverbund besonders begünstigt.

In einem weiteren Ausführungsbeispiel wird das Gerüst zumindest in einem vorgegebenen Bereich vor Einkleben des Markerelements mit einer Beschichtung enthaltend eine pharmazeutisch aktive Substanz versehen.

Hierbei wird unter einer "pharmazeutisch aktiven Substanz" (oder therapeutisch aktive oder wirksame Substanz) ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom Menschen oder tierischen Körper erzeugte Wirkstoffe, wie Insulin, sowie zur Beseitigung oder Unschädlichmachen von Krankheitserregern, Tumoren, Krebszellen oder Körperfremdstoffen Einsatz findet. Bevorzugt wird als pharmazeutisch aktive Substanz eine antiproliferativer Wirkstoff wie beispielsweise Paclitaxel, Sirolimus oder Everolimus verwendet.

Alternativ oder zusätzlich wird das Gerüst zumindest in einem vorgegebenen Bereich nach dem Einkleben des mindestens einen Markerelements mit einer Beschichtung enthaltend eine pharmazeutisch aktive Substanz versehen.

Die obige Aufgabenstellung wird außerdem durch ein scheibenförmiges Markerelement für ein Implantat mit einem Schichtverbund umfassend mindestens eine erste Schicht aufweisend ein röntgenopakes oder radioopakes Material und eine mit der ersten Schicht verbundene und benachbart zu der ersten Schicht angeordnete Klebstoff-Schicht gelöst. Hierbei sind die erste Schicht und die Klebstoff-Schicht mittels eines durch die erste Schicht und die Klebstoff-Schicht durchgehenden Schnitts von dem Halbzeug getrennt.

Durch die Anordnung der Klebstoffschicht auf der röntgenopaken oder radioopaken ersten Schicht und gemeinsame das Ausschneiden des Schichtverbunds aus diesen Schichten aus dem Halbzeug entfällt eine Klebstoffdosierung und -positionierung vor dem Einkleben des Markerelements in das Gerüst bzw. dessen Öffnung.

Bevorzugt ist das scheibenförmige Markerelement aus Tantal, Wolfram, Gold, Platin oder einem anderen Material mit ähnlich hoher Röntgendichte ausgeführt.

Die obige Aufgabenstellung wird ferner gelöst durch ein Gerüst für ein Implantat mit einem oben angegebenen Markerelement, wobei das Markerelement in eine Öffnung (Eyelet) des Gerüsts eingeklebt ist. Das Einklebe-Verfahren wurde oben bereits ausführlich erläutert.

Die obige Aufgabenstellung wird mit den gleichen Vorteilen außerdem durch ein Implantat mit einem oben angegebenen Gerüst gelöst.

Die Erfindung wird nachfolgend anhand von Ausführungsbeispielen und unter Bezugnahme auf die Figuren erläutert. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbezügen.

Es zeigen schematisch:
- Fig. 1: einen Abschnitt eines Gerüsts eines Implantats in einer Ansicht von oben,
- Fig. 2: ein Markerelement in einer Ansicht von oben,
- Fig. 3a-3e: ein erstes Ausführungsbeispiel eines erfindungsgemäßen Verfahrens für die Herstellung eines Markerelements jeweils in einer Schnittdarstellung (Fig. 3a-c) sowie das hieraus hergestellte Markerelement in einer Ansicht von der Seite (Fig. 3d) und in einer perspektivischen Ansicht von der Seite (Fig. 3e),
- Fig. 4a-4d: ein zweites Ausführungsbeispiel eines erfindungsgemäßen Verfahrens für die Herstellung eines Markerelements jeweils in einer Schnittdarstellung (Fig. 4a-b) sowie das hieraus hergestellte Markerelement in einer Ansicht von der Seite (Fig. 4c) und einer perspektivischen Ansicht von der Seite (Fig. 4d),
- Fig. 5: ein zweites Ausführungsbeispiel eines Markerelements in einem Querschnitt, das nicht Teil der Erfindung ist,
- Fig. 6: ein drittes Ausführungsbeispiel eines Markerelements in einer Ansicht von der Seite, das nicht Teil der Erfindung ist,
- Fig. 7a-7d: ein drittes Ausführungsbeispiel eines Verfahrens, das nicht Teil der Erfindung ist, für die Herstellung eines Markerelements (Fig. 7a) sowie das hieraus hergestellte Markerelement in einem Querschnitt (Fig. 7b), einer Ansicht von oben (Fig. 7c) und einer perspektivischen Ansicht von der Seite (Fig. 7d),
- Fig. 8a-8d: ein Ausführungsbeispiel eines erfindungsgemäßen Verfahrens für die Herstellung eines Gerüsts für ein Implantat in einzelnen Schritten jeweils in einer Schnittdarstellung ,
- Fig. 9: ein Werkzeug für die Herstellung des Gerüsts in einer perspektivischen Ansicht von der Seite.
- Fig. 10: ein Ausführungsbeispiel eines Verfahrens, das nicht Teil der Erfindung ist, für die Herstellung eines Gerüsts für ein Implantat mit einem Markerelement nach Fig. 7 in einzelnen Schritten jeweils in einer Schnittdarstellung,
- Fig. 11: ein Ausführungsbeispiel eines Verfahrens, das nicht Teil der Erfindung ist, für die Herstellung eines Gerüsts für ein Implantat mit einem Markerelement nach Fig. 6 in einzelnen Schritten jeweils in einer Schnittdarstellung sowie
- Fig. 12: ein Ausführungsbeispiel analog zu Fig. 11, wobei hier die Öffnung für das Markerelement als Tasche ausgestaltet ist.

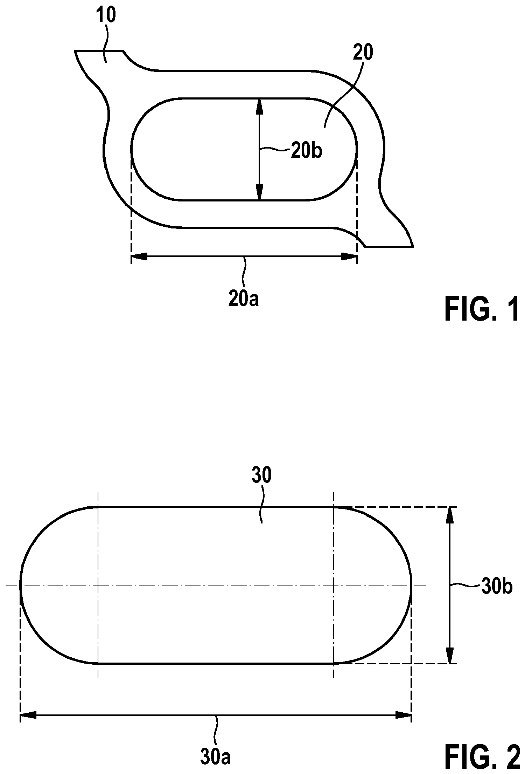

In Fig. 1 ist ein Abschnitt eines Gerüsts 10 eines erfindungsgemäßen Implantats in Form eines medizinischen Stents, beispielsweise bestehend aus der degradierbaren Magnesiumlegierung WE43, dargestellt. Das Bild zeigt eine beispielsweise am distalen oder proximalen Ende des Gerüsts 10 angeordnete durchgehende Öffnung (im Folgenden Eyelet) 20 mit einer elliptischen Grundform. Vorzugsweise sind am distalen und/oder am proximalen Ende des Gerüsts 10 des Implantats jeweils ein Eyelet 20 oder drei um 120° versetzte Eyelets 20 als Bestandteile des Gerüsts, beispielsweise an einer Strebe (Strut), vorgesehen. Hierbei ist das Gerüst vorzugsweise als hohlzylinderförmiges Gitter mit einer Vielzahl von Streben ausgebildet. Die Maße des Eyelets 20 betragen beispielsweise 800 µm (Abmessung 20a in Fig. 1) x 350 µm (Abmessung 20b in Fig. 1).

In dem Eyelet 20 kann ein röntgenopakes Markerelement 30 (siehe Fig. 2) angeordnet werden, das, wie nachfolgend beschrieben wird, mittels einer Klebstoff-Schicht an dem Eyelet 20 befestigt werden kann.

Das in dem Markerelement 30 verwendete röntgenopake Material kann beispielsweise überwiegend aus Tantal (z.B. mit einer Reinheit von 99.9%) oder einer Tantallegierung bestehen. Die Dicke des Markerelements 30 beträgt beispielsweise 100 µm. Die Wanddicke des Gerüsts 10 kann beispielsweise 100 µm betragen. Die Abmessungen des Markers betragen z.B. 750 µm (Abmessung 30a in Fig. 2) x 300 µm (Abmessung 30b in Fig. 2).

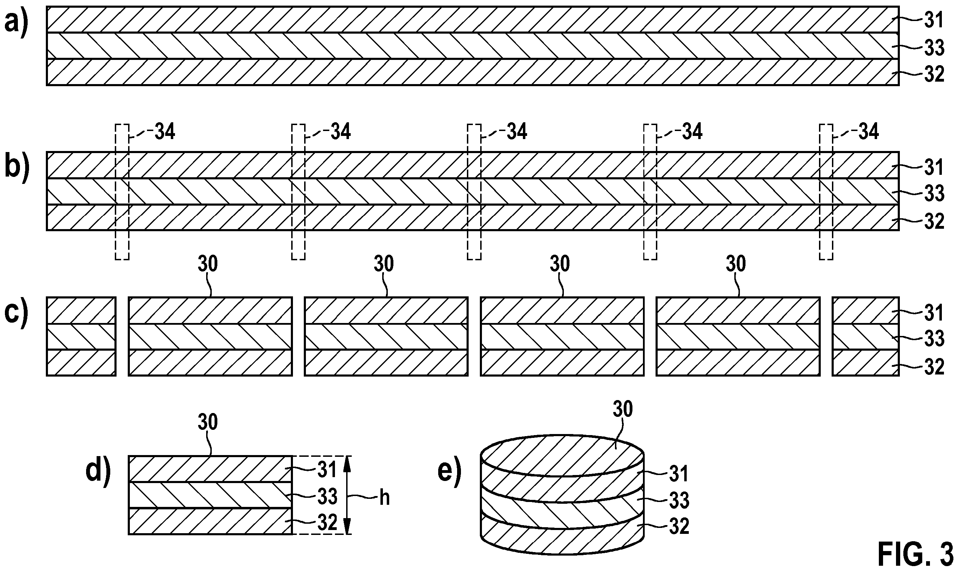

In dem in Fig. 3 gezeigten ersten Ausführungsbeispiel für die Herstellung eines Markerelements 30 wird zunächst ein Halbzeug in Form einer folienartigen ersten Schicht 31 aus dem röntgenopaken oder radioopaken Material, beispielsweise Tantal (z.B. mit einer Reinheit von 99.9%) oder eine Tantallegierung, mit einer Klebstoff-Beschichtung 33, beispielsweise aus einem TPE, versehen. Die Klebstoff-Beschichtung besteht z.B. aus Polyurethan gelöst in Dimethylformamid und weist eine Dicke von ca. 0.025 mm auf. Auf die Klebstoff-Beschichtung 33 wird anschließend auf der der ersten Schicht 31 gegenüber liegenden Seite eine zweite Schicht 32 aus dem röntgenopaken oder radioopaken Material, beispielsweise Tantal oder eine Tantallegierung, aufgebracht. Der hieraus resultierende Schichtverbund ist in Fig. 3a dargestellt.

Im zweiten Schritt werden nun einzelne Markerelemente 30 mittels eines Schneidverfahrens, beispielsweise mittels Laserschneiden oder einem anderen mechanischen Schneidverfahren, aus dem Schichtverbund ausgeschnitten. Das Schneiden ist in Fig. 3b durch gestrichelte Linien 34 dargestellt. Der Schnitt verläuft dabei senkrecht zu den Schichten 31, 32, 33 des Schichtverbunds. Die so vereinzelten Markerelemente 30 weisen jeweils einen Schichtverbund mit drei Schichten auf, wobei die Klebstoff-Schicht 33 sandwich-artig zwischen der ersten Schicht 31 und der zweiten Schicht 32 mit dem röntgenopaken oder radioopaken Material angeordnet ist. Dies ist auch in den Figuren 3d und 3e, in denen jeweils ein einzelnes Markerelement 30 dargestellt ist, ersichtlich.

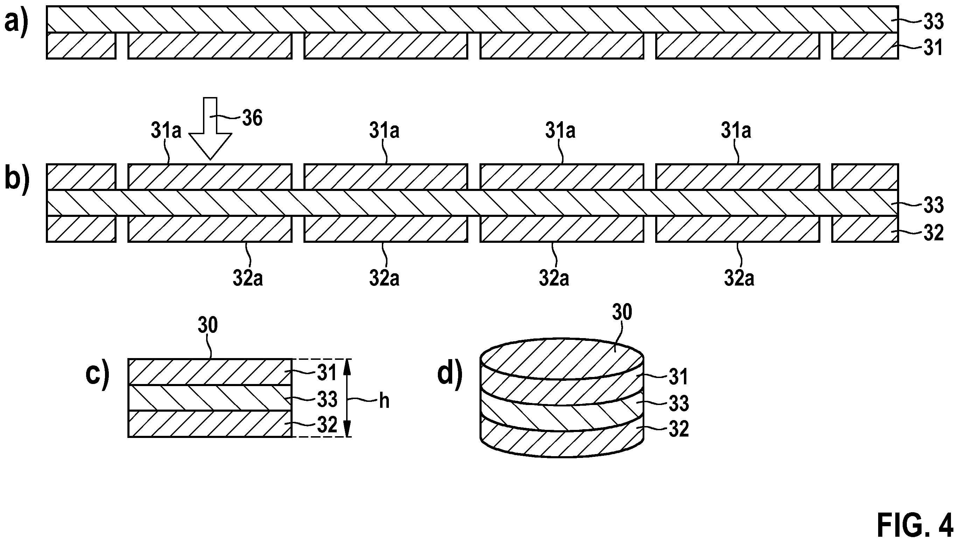

Alternativ kann ein Halbzeug in Form einer folienartigen ersten Schicht 31 mit einem röntgenopaken oder radioopaken Material mit teilweise ausgeschnittenen Abschnitten 3 1a für das Markerelement einseitig mit einer Klebstoff-Schicht 33 (beispielsweise bestehend aus einem TPE) versehen werden (siehe Fig. 4a). Anschließend wird auf der der ersten Schicht 31 gegenüber liegenden zweiten Seite der Klebstoff-Schicht 33 ein weiteres Halbzeug in Form einer folienartigen zweiten Schicht 32 mit einem röntgenopaken oder radioopaken Material mit teilweise ausgeschnittenen Abschnitten 32a aufgebracht (siehe Fig. 4b). Hierbei kann die Oberfläche der Schichten 31a und 32a vor dem Aufbringen der Klebstoff-Beschichtung passiviert worden sein. Eine derartige Passivierung kann beispielsweise durch eine plasmaelektrolytische Behandlung (plasmaelektrolytische Oxidation) erfolgen. Die jeweiligen Abschnitte 31a, 32a liegen dabei übereinander Hierbei bedeutet "teilweise ausgeschnitten", dass der jeweilige Abschnitt 31a, 32a noch über mindestens einen (nicht dargestellten) Steg mit dem übrigen Material der jeweiligen Schicht 31, 32 verbunden ist. Anschließend wird, wie in Fig. 4b dargestellt, mittels einer Kraft in Richtung senkrecht zu den Schichten 31, 32, 33 (siehe Pfeil 36) das Markerelement vereinzelt (heraus gedrückt), indem der mindestens eine Steg der Abschnitte 31a, 32a durchtrennt wird. Die dazwischen angeordnete Klebstoff-Schicht 33 wird ebenfalls durchtrennt. Das Vereinzeln kann auch mittels Laserschneiden oder einem anderen mechanischen Schneidverfahren erfolgen. Das einzelne Markerelement 30, das in den Figuren 4c und 4d dargestellt ist, weist den gleichen Aufbau auf wie ein gemäß Fig. 3 hergestelltes Markerelement 30.

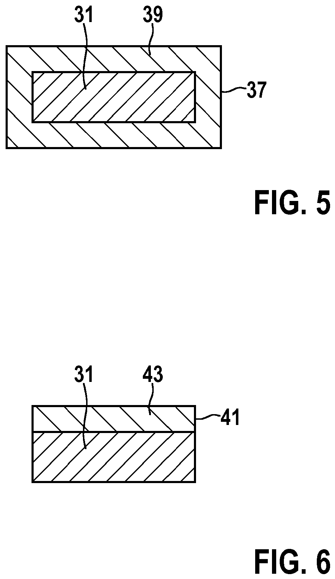

Fig. 5 zeigt ein Markerelement 37 mit einer plättchenförmigen ersten Schicht 31 bestehend aus einem röntgenopaken oder radioopaken Material, beispielsweise Tantal oder einer Tantallegierung, das in einem bereits geschnittenen (vereinzelten) Zustand auf seiner gesamten Oberfläche mit einer Klebstoff-Schicht 39 versehen wurde. Eine solche Beschichtung kann beispielsweise mittels Tauchen in eine entsprechende Lösung oder mittels Besprühen im Schüttgut bestehend aus einer Vielzahl von plättchenförmigen ersten Schichten 31 realisiert werden.

Fig. 6 zeigt ein Markerelement 41 mit einer plättchenförmigen ersten Schicht 31 bestehend aus einem röntgenopaken oder radioopaken Material, beispielsweise Tantal oder einer Tantallegierung, das auf einem Teil seiner Oberfläche mit einer Klebstoff-Beschichtung 43 versehen wurde.

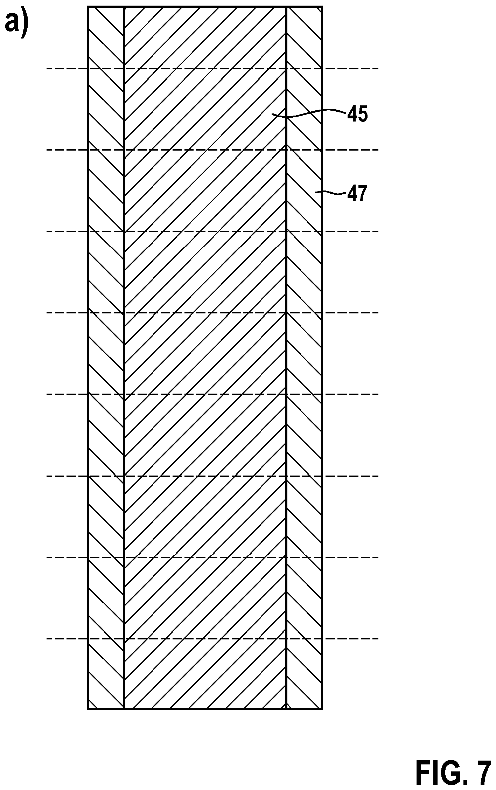

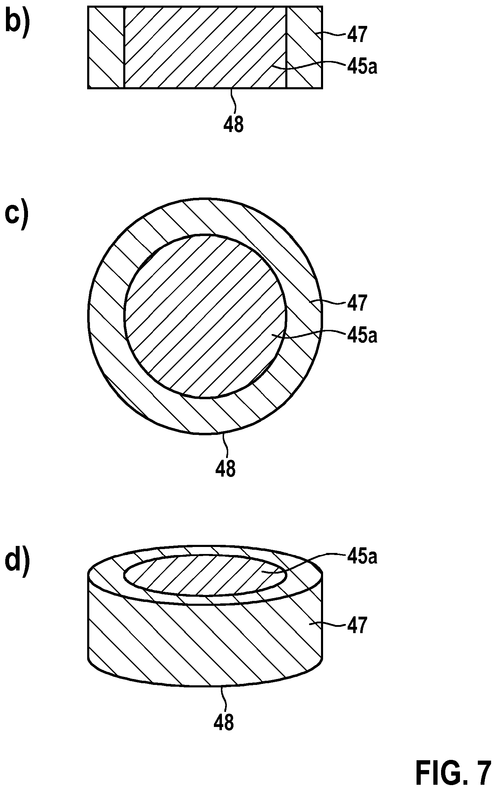

Fig. 7 zeigt ein Verfahren zur Herstellung eines Markerelements, bei dem ein zylinderförmiger Draht 45 bestehend aus dem röntgenopaken oder radioopaken Material, beispielsweise Tantal oder eine Tantallegierung, zunächst auf seiner Mantelfläche mit einer Klebstoff-Schicht 47, insbesondere einem TPE-Material, versehen wurde. Hierbei kann die Oberfläche des Drahts 45 vor dem Aufbringen der Klebstoff-Beschichtung 47 passiviert worden sein.

Anschließend wird der Draht in eine Richtung quer (z.B. senkrecht) zu seiner Längsachse in einzelne Markerelemente 48 zerschnitten. Der Verlauf der Schnitte ist in Fig. 7a mit gestrichelten Linien 49 veranschaulicht. Das Schneiden kann beispielsweise mittels Laserschneiden oder anderen bekannten Schneidverfahren erfolgen. Die hieraus resultierenden Markerelemente 48 sind in den Figuren 7b bis 7d in verschiedenen Ansichten dargestellt. Die Markerelemente 48 zeichnen sich dadurch aus, dass sie ein zylinderförmiges Plättchen 45a aufweisen, das an seinem Umfang mit der Klebstoff-Beschichtung 47 versehen sind (siehe Fig. 7b bis 7d).

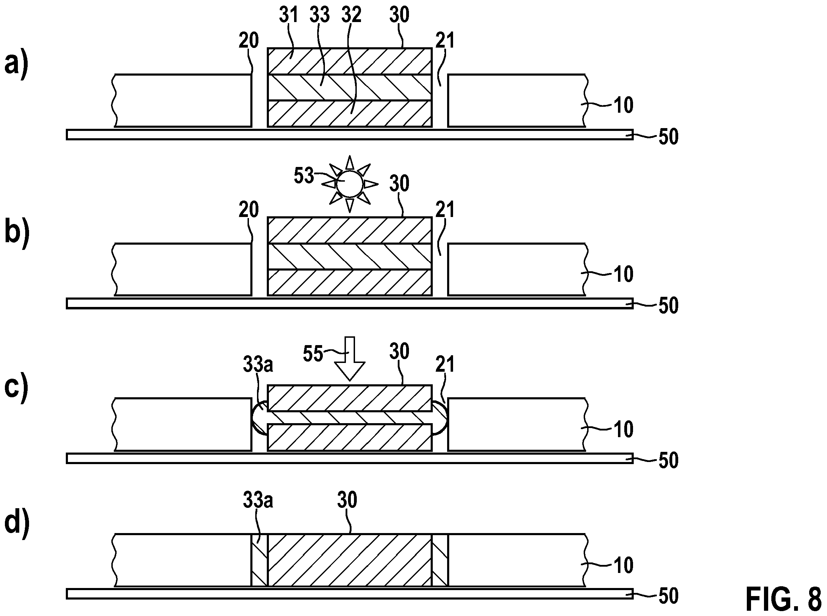

Anhand von Fig. 8 wird nun erläutert, wie ein gemäß Fig. 3 oder 4 hergestelltes Markerelement 30 in eine Öffnung (Eyelet) 20 eines Gerüsts 10 eingebracht und mit diesem verbunden wird.

Vor dem Einlegen des Markerelements 30 in das Eyelet 20 wird das mit dem mindestens einen Markerelement zu bestücken Gerüst 10 auf einen Dorn 50 aufgefädelt. Dies bedeutet, dass das im wesentlichen hohlzylinderförmigen Gerüst 10 auf einen zylinderförmigen Dorn 50 derart gesteckt wird, dass der Dorn 50 das gesamte innere Volumen des hohlzylinderförmigen Gerüsts 10 ausfüllt und dass der Dorn 50 mit seiner Mantelfläche direkt an der Innenseite der Streben des Gerüsts 10 anliegt.

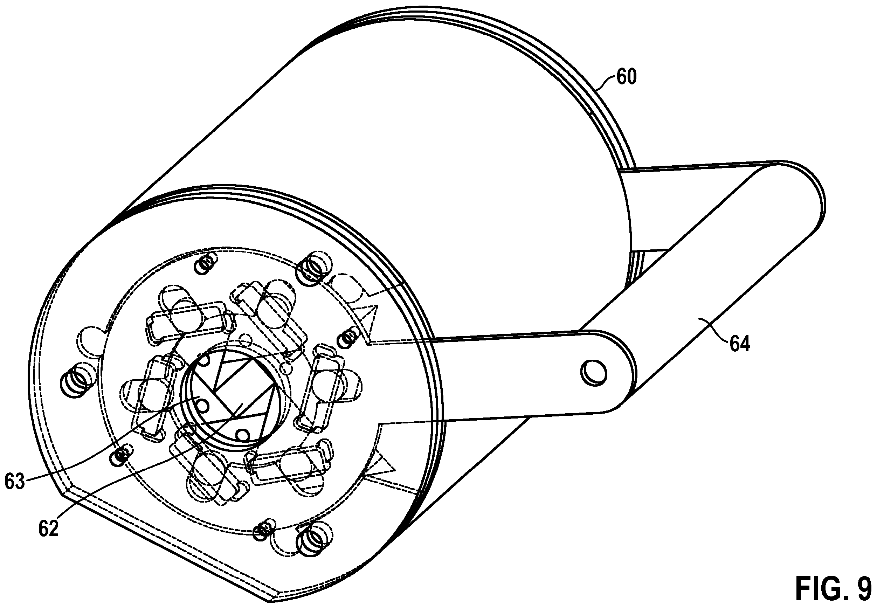

Nachdem das Gerüst 10 auf den Dorn 50 gesteckt wurde, wird nun das Markerelement 30 in ein entsprechendes Eyelet 20 eingelegt (siehe Fig. 8a). Aus der Figur ist ersichtlich, dass zwischen der Mantelfläche des Markerelements 30 und dem Innenrand der Öffnung 20 ein Klebespalt 21 dadurch gebildet wird, dass die äußeren Abmessungen des Markerelements 30 geringfügig kleiner sind als die inneren Abmessungen der Öffnung 20. Anschließend wird unter Einwirkung einer entsprechenden Wärmequelle 53 das Markerelement 30 erwärmt, sodass die Klebstoff-Schicht 33 erweicht wird. Dies ist in Fig. 8b gezeigt. Die Fig. 8c veranschaulicht, dass in dem folgenden Schritt beispielsweise mittels eines in Fig. 9 dargestellten Crimpwerkzeugs 60 das in seiner Höhe h etwas über das Eyelet 20 hinausragende Markerelement 30 zusammengepresst wird. Die Richtung der Presskraft ist eine Richtung quer (z.B. senkrecht) zu dem Verlauf der Schichten bzw. der Längsachse des Gerüsts 10. Die Richtung der Kraft ist in Fig. 8c mit einem Pfeil 55veranschaulicht.

Durch das Einleiten der mechanischen Kraft werden die beiden Schichten 31, 32 des Markerelements 30 zusammengedrückt, so dass für den erweichten Klebstoff der Klebstoff-Schicht das verfügbare Volumen reduziert wird. Folglich wird der erweichte Klebstoff 33a aus der Klebstoff-Schicht 33 seitlich aus dem Markerelement 30 herausgedrückt, sodass der Klebstoff in das leere Volumen des Klebespalts 21 zwischen dem Markerelement 30 und dem inneren Rand des Eyelets 20 des Gerüsts 30 hineinfließt und sich mit dem Innenrand des Eyelets 20 verbindet. Hierdurch kann mittels eines automatisch durchführbaren Verfahrens eine gute Anbindung des Markerelements 30 mittels Klebstoff an das Gerüst 10 erreicht wird. Der Klebstoff 33a füllt den Klebespalt 21 vollständig aus. Dieser Zustand ist in Fig. 8d gezeigt.

Das in Fig. 9 gezeigte Crimpwerkzeug 60 besitzt eine zylinderförmige Öffnung 62, in die das auf den Dorn 50 aufgefädelte Gerüst 10 mit dem noch nicht vollständig befestigten Markerelement 30 eingeführt wird. Um den Umfang der Öffnung 62 sind eine Reihe von Bakken 63 angeordnet, die beim Herunterdrücken des mit den Bakken 63 verbundenen Griffs 64 so geführt werden, dass sich der Durchmesser der Öffnung 62 verkleinert. Hierdurch kann die oben beschriebene Kraft senkrecht zu den Schichten des Markerelements 30 auf das Markerelement 30 aufgebracht werden, um zum Verkleben mit dem Eyelet 20 den Klebstoff 33a aus der Klebstoff-Schicht 33 seitlich herauszudrücken.

Nach dem Pressen in dem Crimpwerkzeug 60 und Aushärten durch Abkühlen des Klebstoffs 33a wird das Gerüst 10 wieder aus dem Crimpwerkzeug 60 entnommen. Anschließend kann der Dorn 50 aus dem Gerüst 10 entfernt werden.

Mittels eines einfachen und einfach zu automatisierenden Verfahrens kann folglich eine dauerhafte Verbindung zwischen Markerelement 30 und Gerüst 10 geschaffen werden, bei dem die Dosierung des Klebstoffs und seine Aufbringung unproblematisch und genau bewerkstelligt werden kann.

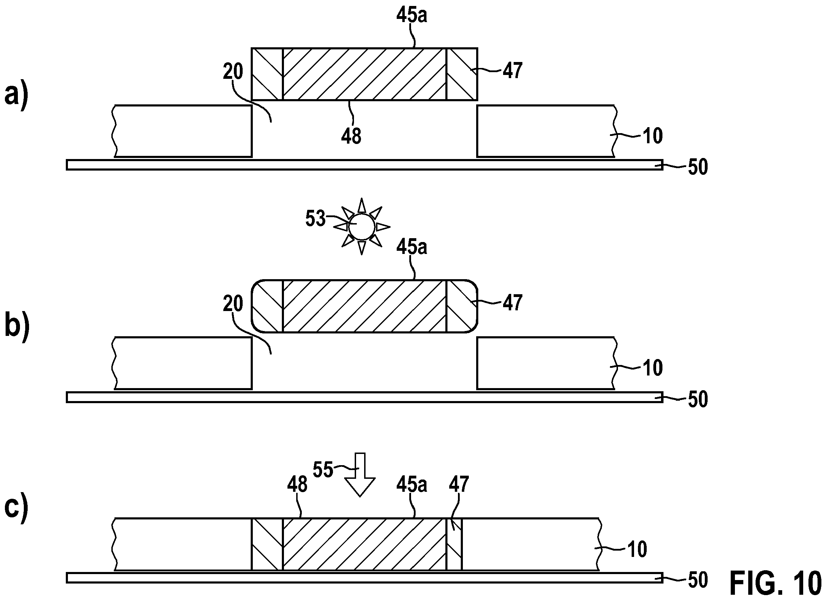

Das Montieren eines Markers 48 (Fig. 10) unterscheidet sich prinzipiell nicht von einem Marker 30. Der Klebstoff befindet sich schon am Umfang des Markers. Durch das Verflüssigen des Klebstoffes vor dem Einpressen des Markers 48 können Formtoleranzen vom Eyelet ausgeglichen werden. Hier ist entsprechend ein kleinerer Klebespalt als bei der Ausgestaltung nach Fig. 8 vorgesehen. Der erwärmte Kleber füllt automatisch den Spalt zwischen Marker 48 und Stent 10.

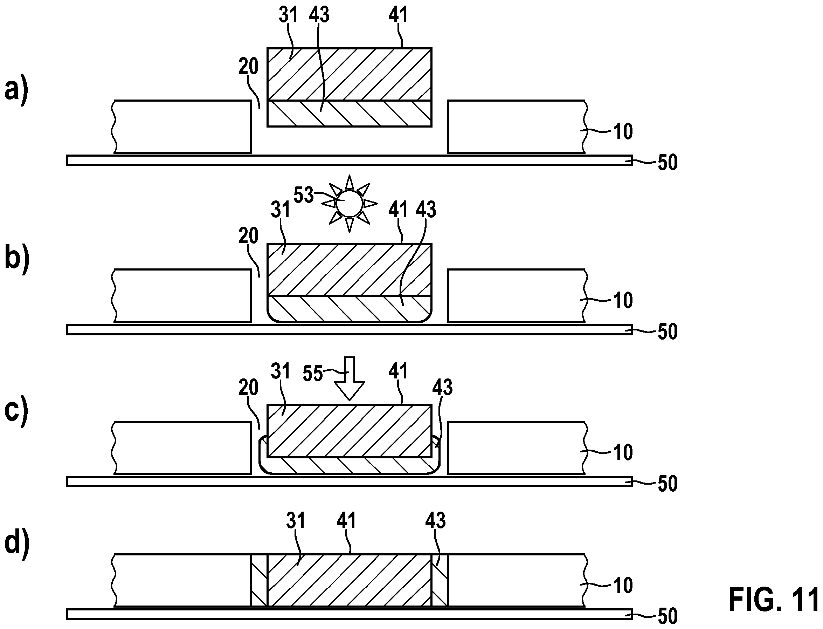

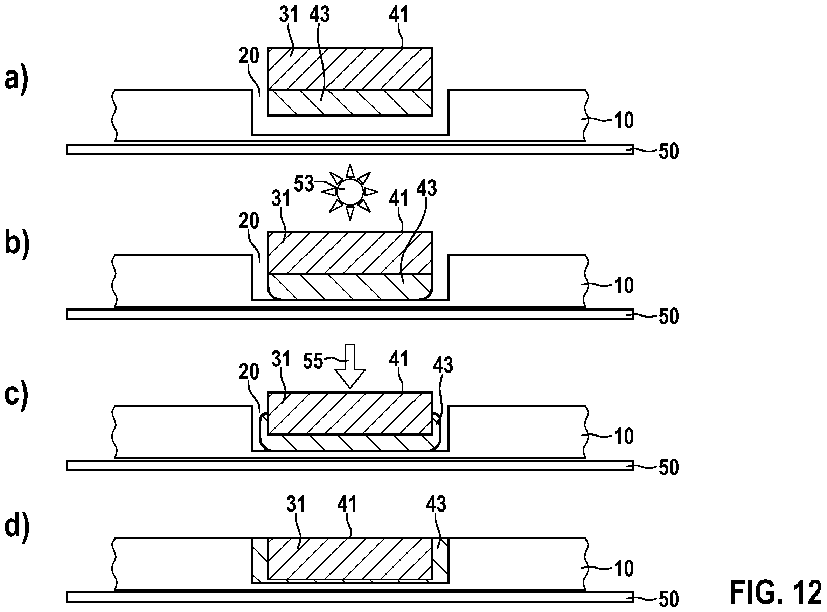

In Fig 11 und 12 ist das Montieren eines Markers 41 gezeigt. Insbesondere wenn das Eyelet nicht als durchgängiges Öffnung, sondern als Tasche ausgearbeitet ist, ist diese Variante vorteilhaft (siehe Fig. 12).

Generell wurden im Rahmen der Zeichnung gleiche Bauteile mit der gleichen Bezugsziffer versehen.

## Patentansprüche

1. Verfahren zur Herstellung eines Markerelements (30, 48) für ein Implantat aus einem schichtförmigen Halbzeug aufweisend ein röntgenopakes oder radioopakes Material, wobei auf oder an mindestens einer Seite des Halbzeugs (31, 32) oder einer Vielzahl von nebeneinander angeordneten Abschnitten (31a, 32a) des Halbzeugs jeweils eine Klebstoff-Schicht (33, 47) befestigt wird, so dass zumindest abschnittsweise ein schichtförmiger Schichtverbund gebildet wird, und dass anschließend eine Vielzahl von scheibenförmigen Markerelementen (30, 48) durch einen entsprechenden Schnitt oder eine entsprechende Durchtrennung in eine Richtung quer, vorzugsweise senkrecht, zu den Schichten des Schichtverbunds aus dem Schichtverbund ausgeschnitten oder von dem Schichtverbund abgetrennt werden, **dadurch gekennzeichnet, dass**,
- eine durchgehende erste Schicht (31) oder eine Vielzahl von Abschnitten (31a) einer ersten Schicht des Halbzeugs auf einer ersten Seite der Klebstoff-Schicht (33) sowie
- eine durchgehende zweite Schicht (32) oder eine Vielzahl von Abschnitten (32a) einer zweiten Schicht des Halbzeugs auf einer zweiten, der ersten Seite gegenüber liegenden Seite der Klebstoff- Schicht (33)
angeordnet und an dieser befestigt werden, so dass diese zumindest abschnittsweise den Schichtverbund bilden.

2. Verfahren nach Anspruch 1, **dadurch gekennzeichnet, dass** die Klebstoff-Schicht (33, 47) ein thermoplastisches Elastomer aufweist.

3. Verfahren zur Herstellung eines Gerüsts (10) für ein Implantat mit einem scheibenförmigen Markerelement (30, 37, 41, 48) aufweisend einen schichtförmigen Schichtverbund mit mindestens einer Schicht aufweisend ein röntgenopakes oder radioopakes Material und einer Klebstoff-Schicht (33, 39, 43, 47), wobei das Markerelement (30, 37, 41, 48) in eine Öffnung (20) des Gerüsts (10) eingebracht und das Markerelement (30, 37, 41, 48) mittels einer Wärmequelle (53) derart erwärmt wird, dass die Klebstoff-Schicht (33, 39, 43, 47) des Markerelements (30, 37, 41, 48) erweicht oder verflüssigt wird, sodass der Klebstoff (33a) der Klebstoff-Schicht (33, 39, 43, 47) eine Klebstoff-Verbindung mit der Innenfläche der Öffnung (20) bewirkt, **dadurch gekennzeichnet dass** der Schichtverbund mindestens eine erste Schicht (31) und eine zweite Schicht (32), jeweils aufweisend ein röntgenopakes oder radioopakes Material, und eine dazwischen liegende Klebstoff-Schicht (33) aufweist, wobei das Markerelement (30) nach dem Einbringen in eine Öffnung (20) des Gerüsts (10) derart mit einer Druckkraft in eine Richtung quer zu den Schichten des Schichtverbunds beaufschlagt wird, dass der Klebstoff (33a) an der Seitenfläche des Markerelements (30) austritt und eine Klebstoff-Verbindung mit der Innenfläche der Öffnung (20) bewirkt.

4. Verfahren nach Anspruch 3, **dadurch gekennzeichnet, dass** das Markerelement (30) vor oder während der Beaufschlagung des Markerelements mit der Druckkraft die Wärmequelle (53) derart erwärmt wird, dass die Klebstoff-Schicht (33) des Markerelements (33) erweicht oder verflüssigt wird.

5. Verfahren nach einem der Ansprüche 3 bis 4, **dadurch gekennzeichnet, dass** das Gerüst (10) zumindest in einem vorgegebenen Bereich vor dem Einkleben des Markerelements (30, 48) in die Öffnung (20) des Gerüsts (10) mit einer Beschichtung enthaltend eine pharmazeutisch aktive Substanz versehen wird.

6. Verfahren nach einem der Ansprüche 3 bis 4, **dadurch gekennzeichnet, dass** das Gerüst (10) zumindest in einem vorgegebenen Bereich nach dem Einkleben des Markerelements (30, 48) in die Öffnung (20) des Gerüsts (10) mit einer Beschichtung enthaltend eine pharmazeutisch aktive Substanz versehen wird.

7. Scheibenförmiges Markerelement (30) für ein Implantat mit einem schichtförmigen Schichtverbund umfassend mindestens eine erste Schicht (31) aufweisend ein röntgenopakes oder radioopakes Material und eine Klebstoff-Schicht (33), **dadurch gekennzeichnet dass** das Markerelement eine zweite Schicht (32) aufweisend ein röntgenopakes oder radioopakes Material aufweist, und dass die Klebstoffschicht (33) zwischen der ersten Schicht (31) und der zweiten Schicht (32) liegt.

8. Gerüst (10) für ein Implantat mit einem Markerelement (30) nach Anspruch 7, **dadurch gekennzeichnet, dass** das Markerelement (30) in eine Öffnung (20) des Gerüsts (10) eingeklebt ist.

9. Implantat mit einem Gerüst (10) nach Anspruch 8.

## Claims

1. A method for producing a marker element (30, 48) for an implant formed from a layered semi-finished product comprising an X-ray opaque or radiopaque material, wherein an adhesive layer (33, 47) is attached on or to at least one side of the semi-finished product (31, 32) or a plurality of adjacently arranged portions (31a, 32a) of the semi-finished product, so that a layered layer composite is formed at least in portions, and a plurality of disc-like marker elements (30, 48) are then cut from the layer composite or separated from the layer composite by a corresponding cut or a corresponding severing in a direction transverse, preferably perpendicular, to the layers of the layer composite, **characterised in that**
- a continuous first layer (31) or a plurality of portions (31a) of a first layer of the semi-finished product is arranged on and attached to a first side of the adhesive layer (33) and also
- a continuous second layer (32) or a plurality of portions (32a) of a second layer of the semi-finished product is arranged on and attached to a second side of the adhesive layer (33), opposite the first side,
so that these form the layer composite at least in portions.

2. The method according to claim 1, **characterised in that** the adhesive layer (33, 47) comprises a thermoplastic elastomer.

3. A method for producing a scaffold (10) for an implant, said scaffold having a disc-shaped marker element (30, 37, 41, 48) comprising a layered layer composite with at least one layer comprising an X-ray opaque or radiopaque material and an adhesive layer (33, 39, 43, 47), wherein the marker element (30, 37, 41, 48) is introduced into an opening (20) of the scaffold (10) and the marker element (30, 37, 41, 48) is heated by means of a heat source (53) in such a way that the adhesive layer (33, 39, 43, 47) of the marker element (30, 37, 41, 48) softens or liquefies, so that the adhesive (33a) of the adhesive layer (33, 39, 43, 47) brings about an adhesive connection to the inner face of the opening (20), **characterised in that** the layer composite has at least one first layer (31) and one second layer (32), each comprising an X-ray opaque or radiopaque material and an adhesive layer (33) arranged in between, wherein the marker element (30), following the introduction into an opening (20) of the scaffold (10), is acted on by a compressive force in a direction transverse to the layers of the layer composite, in such a way that the adhesive (33a) exits at the side face of the marker element (30) and brings about an adhesive connection to the inner face of the opening (20).

4. The method according to claim 3, **characterised in that** the marker element (30) is heated by the heat source (53) before or during the application of the compressive force to the marker element, in such a way that the adhesive layer (33) of the marker element (33) is softened or liquefied.

5. The method according to any one of claims 3 to 4, **characterised in that** the scaffold (10) is provided, at least in a predefined area, with a coating containing a pharmaceutically active substance before the marker element (30, 48) is adhesively bonded in the opening (20) of the scaffold (10).

6. The method according to any one of claims 3 to 4, **characterised in that** the scaffold (10), at least in a predefined area, is provided with a coating containing a pharmaceutically active substance after the marker element (30, 48) has been adhesively bonded in the opening (20) of the scaffold (10).

7. A disc-shaped marker element (30) for an implant, said marker element having a layered layer composite comprising at least a first layer (31) comprising an X-ray opaque or radiopaque material and an adhesive layer (33), **characterised in that** the marker element comprises a second layer (32) comprising an X-ray opaque or radiopaque material, and **in that** the adhesive layer (33) lies between the first layer (31) and the second layer (32).

8. A scaffold (10) for an implant, said scaffold comprising a marker element (30) according to claim 7, **characterised in that** the marker element (30) is adhesively bonded in an opening (20) of the scaffold (10).

9. An implant having a scaffold (10) according to claim 8.

## Revendications

1. Procédé de fabrication d'un élément de marquage (30, 48) pour un implant à base d'un semi-produit en forme de couche présentant un matériau opaque aux rayons X ou radio-opaque, dans lequel une couche d'adhésif (33, 47) est respectivement fixée sur ou au niveau d'au moins un côté du semi-produit (31, 32) ou d'une multiplicité de segments (31a, 32a) disposés les uns à côté des autres du produit semi-fini, de sorte qu'au moins par endroits un composite en couches en forme de couche est formé, et qu'ensuite une multiplicité d'éléments de marquage (30, 48) en forme de disques sont découpés par une découpe correspondante ou une séparation correspondante dans une direction perpendiculaire, de préférence verticale, par rapport aux couches du composite en couches à partir du composite en couches ou sont séparés du composite en couches,
**caractérisé en ce**
**qu'**une première couche (31) traversante ou une multiplicité de segments (31a) d'une première couche du semi-produit est disposée sur un premier côté de la couche d'adhésif (33), ainsi
**qu'**une deuxième couche (32) traversante ou une multiplicité de segments (32a) d'une deuxième couche du semi-produit est disposée sur un deuxième côté de la couche d'adhésif (33) situé en face du premier côté
et sont fixées à ceux-ci de sorte que ceux-ci forment au moins par endroits le composite en couches.

2. Procédé selon la revendication 1, **caractérisé en ce que** la couche d'adhésif (33, 47) présente un élastomère thermoplastique.

3. Procédé de fabrication d'une structure (10) pour un implant avec un élément de marquage (30, 37, 41, 48) en forme de disque présentant un composite en couches en forme de couche avec au moins une couche présentant un matériau opaque aux rayons X ou radio-opaque et une couche d'adhésif (33, 39, 43, 47), où l'élément de marquage (30, 37, 41, 48) est introduit dans un orifice (20) de la structure (10) et l'élément de marquage (30, 37, 41, 48) est chauffé au moyen d'une source de chaleur (53) de telle manière que la couche d'adhésif (33, 39, 43, 47) de l'élément de marquage (30, 37, 41, 48) se ramollit ou devient liquide de sorte que l'adhésif (33a) de la couche d'adhésif (33, 39, 43, 47) provoque une liaison adhésive avec la surface intérieure de l'orifice (20),
**caractérisé en ce que** le composite en couches présente au moins une première couche (31) et une deuxième couche (32), présentant respectivement un matériau opaque aux rayons X ou radio-opaque et présente une couche d'adhésif (33) se situant entre elles, dans lequel l'élément de marquage (30), après l'introduction dans un orifice (20) de la structure (10), est soumis à une force de pression dans une direction perpendiculaire aux couches du composite en couches que l'adhésif (33a) sort au niveau de la surface latérale de l'élément de marquage (30) et provoque une liaison adhésive avec la surface intérieure de l'orifice (20.

4. Procédé selon la revendication 3, **caractérisé en ce que** l'élément de marquage (30), avant ou pendant la soumission de l'élément de marquage, avec la force de pression, la source de chaleur (53) est chauffée de telle manière que la couche d'adhésif (33) de l'élément de marquage (33) se ramollit ou devient liquide.

5. Procédé selon l'une des revendications 3 à 4, **caractérisé en ce que** la structure (10) est munie au moins dans une zone prédéfinie avant le collage de l'élément de marquage (30, 488) dans l'orifice (20) de la structure (10) d'un revêtement contenant une substance pharmaceutiquement active.

6. Procédé selon l'une des revendications 3 à 4, **caractérisé en ce que** la structure (10) est munie au moins dans une zone prédéfinie après le collage de l'élément de marquage (30, 488) dans l'orifice (20) de la structure (10) d'un revêtement contenant une substance pharmaceutiquement active.

7. Elément de marquage (30) en forme de disque pour un implant avec un composite en couches en forme de couche comprenant au moins une première couche (31) présentant un matériau opaque aux rayons X ou radio-opaque et une couche d'adhésif (33), **caractérisé en ce que** l'élément de marquage présente une deuxième couche (32) présentant un matériau opaque aux rayons X ou radio-opaque et que la couche d'adhésif (33) se situe entre la première couche (31) et la deuxième couche (32).

8. Structure (10) pour un implant avec un élément de marquage (30) selon la revendication 7, **caractérisé en ce que** l'élément de marquage (30) est collé dans un orifice (20) de la structure (10).

9. Implant avec une structure (10) selon la revendication 8.
